(19) Europäisches Patentamt / European Patent Office / Office européen des brevets

(11) **EP 3 810 315 B1**

(12) **EUROPEAN PATENT SPECIFICATION**

(45) Date of publication and mention
of the grant of the patent:
**26.11.2025 Bulletin 2025/48**

(21) Application number: **19763090.8**

(22) Date of filing: **20.06.2019**

(51) International Patent Classification (IPC):
**C09K 23/14** (2022.01)     **A61K 8/55** (2006.01)
**A61Q 19/00** (2006.01)

(52) Cooperative Patent Classification (CPC):
**A61K 8/553; A61K 8/0295; A61K 8/342;**
**A61K 8/55; A61Q 19/00;** C09K 23/14

(86) International application number:
**PCT/IB2019/055215**

(87) International publication number:
**WO 2019/244097 (26.12.2019 Gazette 2019/52)**

(54) **PROCESS FOR FORMATION OF EMULSION CONTAINING LIQUID CRYSTAL STRUCTURE**

VERFAHREN ZUR BILDUNG EINER EMULSION MIT FLÜSSIGKRISTALLSTRUKTUR

PROCÉDÉ DE FORMATION D'UNE ÉMULSION CONTENANT UNE STRUCTURE DE CRISTAUX LIQUIDES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **22.06.2018 US 201862688714 P**

(43) Date of publication of application:
**28.04.2021 Bulletin 2021/17**

(73) Proprietor: **Kenvue Brands LLC**
**Summit, NJ 07901 (US)**

(72) Inventors:
• **TANG, Xudong**
**Skillman, New Jersey 08558 (US)**
• **PERNA, Christine**
**Skillman, New Jersey 08558 (US)**

(74) Representative: **Carpmaels & Ransford LLP**
**One Southampton Row**
**London WC1B 5HA (GB)**

(56) References cited:
**WO-A1-2017/074892    KR-A- 20020 094 478**

• **M. KLACSOVÁ ET AL: "The effect of aliphatic alcohols on fluid bilayers in unilamellar DOPC vesicles - A small-angle neutron scattering and molecular dynamics study", BIOCHIMICA ET BIOPHYSICA ACTA (BBA) - BIOMEMBRANES, vol. 1808, no. 9, 1 September 2011 (2011-09-01), AMSTERDAM, NL, pages 2136 - 2146, XP055644971, ISSN: 0005-2736, DOI: 10.1016/ j.bbamem.2011.04.010**
• **G. PENNICK ET AL: "The effect of an amphiphilic self-assembled lipid lamellar phase on the relief of dry skin", INTERNATIONAL JOURNAL OF COSMETIC SCIENCE., vol. 34, no. 6, 1 September 2012 (2012-09-01), NL, pages 567 - 574, XP055540514, ISSN: 0142-5463, DOI: 10.1111/ j.1468-2494.2012.00749.x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The use of specified homogenizer configuration, i.e., stator and rotor and shear energy density, to form a liquid crystal structure between (1) negatively charged phospholipid and/or phospholipid derivatives; and (2) fatty alcohols.

The invention is defined in the appended claims. The use of potassium cetyl phosphate hydrogenated palm glycerides (Emulsiphos®, Symrise, HLB 13-14) and cetyl alcohol is specifically disclosed.

A liquid crystal structure composition may be formulated using a negatively charged phospholipid (i.e., an anionic phospholipid). The solubility limit of the phospholipid depends upon the total composition of the liquid crystal structure composition and may vary depending on the other ingredients in the composition. As a guideline, the upper limit would be approximately 3.5 weight percent.

Phospholipids, as used herein, refers to molecules consisting of a hydrophilic head and a hydrophobic tail. Phospholipids tend to line up and arrange themselves into two parallel layers, referred as a phospholipid bilayer. Such layer, which makes up cell membranes, is critical to a cell's ability to function. When the phospholipid is negatively charged, it mimics the human skin.

To make the liquid crystal structure composition more like skin, the composition may contain a pH adjustor to maintain the pH of the composition close to that of human skin, i.e. a pH of about 4 to about 5.5, although slightly above and slightly below this pH are contemplated to be within the realm of the invention. In addition, ingredients typically found in skin care compositions, e.g., active ingredients, colorants and fragrances, may be included in the composition of the invention.

The use of an anionic phospholipid or negatively charged phospholipid in the liquid crystal structure composition of the invention plays a role in the beneficial results described above.

Phospholipids differ in size, shape and charge of their polar head groups. They may be anionic, cationic or non-ionic.

Examples of anionic phospholipids include dimyristoylphosphatidylglycerol, dipalmitoylphosphatidylglycerol, distearoylphosphatidylglycerol, dioleoylphosphatidylglycerol, oleoylpalmitoylphosphatidylglycerol, dipalmitoylphosphatidylserine, dioleoylphosphatidylserine, dimyristoylphosphatidylinositol, dipalmitoylphosphatidylinositol, phosphatidylethanolamine, distearoylphosphatidylinositol, dioleoylphosphatidylinositol, dimyristoylphosphatidylserine, and distearoylphosphatidylserine. Of these materials, phosphoglycerides and phosphatidyethanolamine are the preferred phospholipids.

Other examples of phospholipid and/or phospholipid derivatives include, for example, natural phospholipid derivates such as egg PC (egg lecithin), egg PG, soy PC, hydrogenated soy PC, sphingomyelin as natural phospholipids; and synthetic phospholipid derivates such as phosphatidic acid (DMPA, DPPA, DSPA); phosphatidylcholine (DDPC, DLPC, DMPC, DPPC, DSPC, DOPC, POPC, DEPC); phosphatidylglycerol (DMPG, DPPG, DSPG, POPG); phosphatidylethanolamine (DMPE, DPPE, DSPE DOPE); phosphatidylserine (DOPS); PEG phospholipid (mPEG-phospholipid, polyglycerin-phospholipid, functionalized-phospholipid, terminal activated-phospholipid).

As the concentration of phospholipid increases, it becomes more difficult to dissolve and distribute within the composition. If the total phospholipid content exceeds its solubility limit and is incompletely solubilized, it may be present as a separate phase resulting in a tacky feel to the skin following use of the composition.

Fatty alcohols that may be used in the liquid crystal structure composition according to the present invention include any of various alcohols derived from oils and fats (e.g., from plant or animal sources) or synthetic hydrophobic groups. The fatty alcohol comprises from 8 to 34, preferably from 7 to 22 carbon atoms, more preferably 9 to 16 carbon atoms, and even more preferably 11 to 16 carbon atoms. Suitable fatty alcohols may comprise one or more alcohol groups per molecule. According to the invention, the fatty alcohol is cetyl alcohol.

BACKGROUND OF THE INVENTION

Emulsions are the most commonly used delivery system for personal care products, as they impart desired qualities such as skin hydration, skin compatibility, physical characteristics, ease of application, and consumer preference.[1,2]

Recently, emulsions with liquid crystalline structures have been of interest to the industry. These types of emulsions are typically composed of crystalline materials that can swell and thicken water. The crystalline network is stabilized by lamellar bilayers of material that bind water. The structure exhibits viscoelastic properties and shear thinning effect when applied to the skin surface. Additionally, liquid crystalline structures have better application performance than conventional emulsion systems in terms of stability, controlled release and moisturization.[3-5]

WO2017074892 is said to disclose a topical o/w emulsion having moisturizing, and protecting, repairing or restoring the skin lipid barrier of the lips of a mammal, and is said to disclose a topical oil-in- water emulsion composition comprising: (a) a discontinuous oil phase; (b) a continuous aqueous phase comprising water; (c) a thickening agent; (d) at least one lamellar membrane structure comprising (i) an alkyl amphiphilic component, (ii) an ester of a branched fatty acid and a branched fatty alcohol, (iii) a fatty acid, (iv) a fatty alcohol, and optionally (v) a phospholipid; and wherein in use the composition has a water vapor transmission rate of less than 65 g.m-2.hr-1 measured in vitro.

The quality of personal care products containing liquid crystalline structures depends not only on the composition of the emulsion formulation, but also on the manufacturing processes. Energy Density,

$$Ev = \frac{Energy\ input\ E}{homogenized\ volume\ V} / (\text{homogenized volume V})$$ and number of stages of rotor and stators are important elements for homogenization. These attributes greatly influence the minimum droplet size achievable and the molecular association of the materials, which greatly impacts the stability profile and are key factors to ensure robust formation of the liquid crystal structure. Achieving the minimum particle size, which is described as $d = c\,(Ev)^b$, where c and b are constants, D is the particle size, Ev is the energy density, ensures an aesthetic appearance that is acceptable to consumers.

A newly developed chassis (o/w emulsion, see detailed formula in Table 1 below) forms a liquid crystal structure. Challenges were encountered during process development for this chassis. For example, white specks were observed in the product after several days at room temperature.

The inventors determined that a specified homogenizer configuration, including three stages of rotor and stator, resulted in an emulsion containing liquid crystals with good properties.

BRIEF DESCRIPTION OF THE FIGURES

Figure 1 shows (a) product prepared using a comparative method (white specks); and (2) product prepared in accordance with the process of the invention (no white specks).

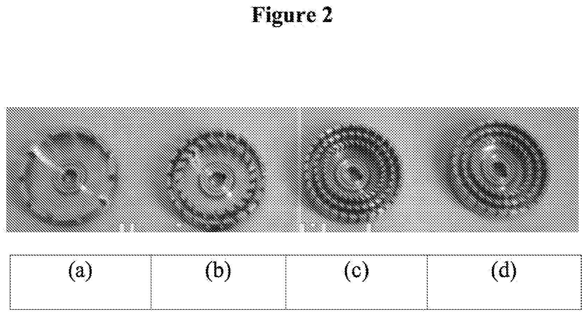

Figures 2(a)-2(d) show different stages of stator and rotor.

Figures 3(a)-3(e) show the appearance of product upon using different homogenizers.

Figure 4 shows a polarized light microscopy image of individual crystals within a speck aggregate.

Figure 5 shows SEM-EDS elemental analysis of white speck in the product.

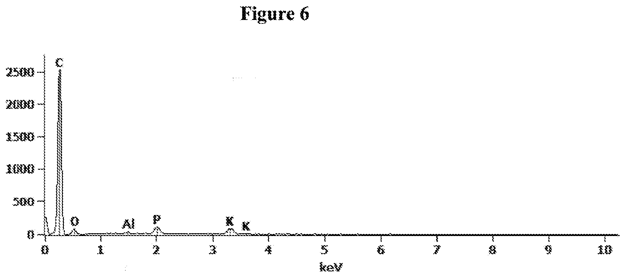

Figure 6 shows SEM-EDS elemental analysis of Emulsiphos®.

Figures 7(a)-7(d) shows FT-IR chromatory graph results.

Figures 8(a)-8(d) shows DSC results.

Figure 9 shows the lamellar liquid crystalline structures of the stratum corneum.

Figure 10 shows shows the lamellar liquid crystalline structures of Emulsiphos® and cetyl alcohol.

DETAILED DESCRIPTION OF THE INVENTION

The invention is defined in the appended claims. Naturally occurring skin lipids and sterols, as well as artificial or natural oils, humectants, emollients, lubricants, etc., may be part of the composition the invention.

An "emollient" is an additive that has the quality of softening or soothing the skin. Emollients are generally complex mixtures of chemical compounds that hold water in the skin after application and help smooth the skin. Emollients increase the skin's hydration (water content) by reducing evaporation. Preferred emollients are cocoglycerides, which are mixtures of mono, di and triglycerides derived from coconut oil.

An "emollient wax" or "wax" is an additive that (1) has the properties of an emollient; (2) is oilbased; (3) is solid at room temperature. A preferred emollient wax is cetyl alcohol, a fatty alcohol - palmitate/ester that is also known as hexadecan-1-ol or palmityl alcohol, available as Lanette®16 from BASF Care Creations, Monheim, Germany. Lanette® 16 is a cetyl alcohol that is used for viscosity regulation in cosmetic and pharmaceutical oil-in-water emulsions. It is a white to light yellowish hydrophilic wax that is supplied in pellets or flakes. This product has a hydroxyl value of 228-234, a hydrocarbon content of max. 0.5%, and a solidification point of 47-50°C. The HLB value of cetyl alcohol is about 15.5. Other examples include petrolatum and silicone-derived ingredients, such as cyclomethicone.

An "emulsifier" is an additive that stabilizes a mixture of two or more liquids that are normally immiscible. An example of an emulsifier is Emulsiphos®, a phospholipid derivative that is a potassium salt of a complex mixture of esters of phosphoric acid available from Symrise GmbH & Co., Holzmiden, Germany.

A "gelling agent" is an additive that can form a polymer gelled composition by crosslinking or neutralization. Gelling agents can also stabilize emulsions, form gels, increase viscosity, etc. Examples of gelling agents include polyacrylate (such as carbomer) and polysaccharide (such as cellulose). A preferred gelling agent is carbomer, which is a polymeric chemical composed of acrylic acid monomers.

The composition according to the present invention is prepared by the claimed process and may further contain the following amounts of the specified ingredients:

an emollient, preferably cocoglyceride, from about >0% to about 10%, preferably from about 2% to about 6%; more preferably from about 3 to about 6%;

an emollient wax, preferably a cetyl alcohol, from about >0% to about 8%, preferably from about 1% to about 4%; more preferably from about 1.5% to about 3%;

an emulsifier, preferably a cetyl phosphate, from about 0.2% to about 1.4%, preferably from about 0.4% to about 1.4%; more preferably from about 0.5% to about 0.6%;

a gelling agent, preferably a carbomer, from about 0.4% to about 0.6%, preferably from about 0.4% to about 0.55%; and

from about 60% to about 90% water.

All percentages (%) are by weight unless otherwise specified herein.

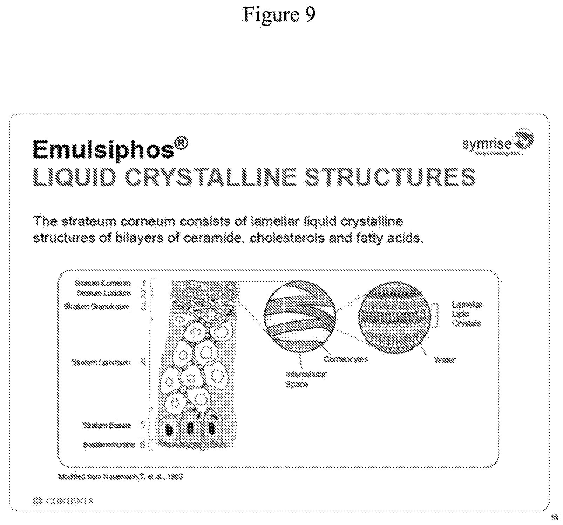

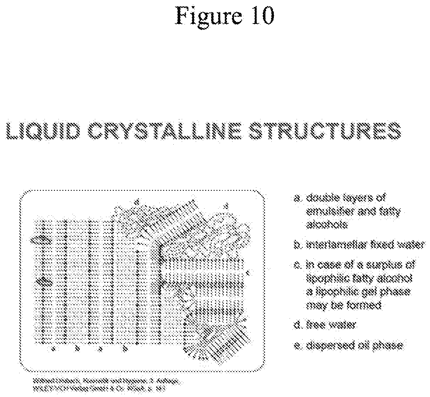

The chassis was developed to achieve similarity of the lamellar structure formed by Emulsiphos®, Symrise, Inc., Branchburg, NJ (potassium cetyl phosphate, hydrogenated palm glycerides) and cetyl alcohol to the lipid phase under the skin. See Figure 9. The structure for this formula can be seen in Figure 10, which shows a polar head for Emulsiphos® and a polar head for cetyl alcohol. The intercalation of the cetyl alcohol (fatty alcohol) disturbs the rigidity of the structure, thus making the product more fluid-like.

From a processing standpoint, the right homogenizer configuration, i.e., stator and rotor and shear energy density, ensured proper formation, i.e., successful "intercalation" of the fatty alcohol to the phospholipid, which resulted in good appearance, stability profile, and skin barrier protection.

Examples

Three stages of rotor and stator have been proposed for this invention to solve the problem. As shown below, through increased residence time and more turbulent mixing, multiple stages enhance the mixing and interaction between the ingredients in the composition.

The process of the invention was used on the formula in Table 1 to obtain the desired properties.

The composition of Table 1 may be prepared following the procedures described below:

In the main vessel introduce water, disodium EDTA and carbomer.
Control good dispersion of carbomer and then heat to 80°C.
Add glycerin and control temperature (80°C).
Add Emulsiphos®, cocoglyceride and cetyl alcohol.
Control that the Emulsiphos® is completely melted: 15 min emulsion phase at 80°C. Neutralize with an aqueous solution of sodium hydroxide, p-anisic acid to a target pH=5.6.
Start cooling down to 35°C.
When temperature reaches 40°C, optionally add ethylhexylglycerin; phenoxyethanol, then corn starch.
Check the good dispersion of purity and pH. Adjust pH if needed with sodium hydroxide to pH=5.6.

Table 1

| US INCI Name | % | Function |
|---|---|---|
| Water | 85.36 2 | Vehicle |

(continued)

| US INCI Name | % | Function |
|---|---|---|
| Glycerin | 5 | Humectant |
| Disodium EDTA | 0.2 | Chelating agent |
| Carbomer | 0.4 | Viscosity Increasing Agent |
| p-Anisic Acid | 0.15 | Masking Agent |
| Sodium Hydroxide | 0.188 | pH Adjuster |
| Potassium Cetyl Phosphate;Hydrogenated Palm Glycerides (Emulsiphos®) | 0.5 | Emulsifier |
| Cetyl Alcohol | 2 | Emollient |
| Cocoglycerides | 4 | Emollient |
| Ethylhexylglycerin; Phenoxyethanol | 0.6 | Preservative |
| Ethylhexylglycerin | 0.2 | Skin Conditioner |
| Zea Mays (Corn) Starch | 1 | Absorbent |
| Water; Pyrus Malus (Apple) Fruit Extract; Citric Acid; Sodium Benzoate; Potassium Sorbate | 0.1 | Skin Conditioning Agent Occlusive |
| Fragrance | 0.3 | Fragrance |

Compositions were prepared on a larger scale in accordance with the process conditions set forth in Table 2 below.

Table 2

| | 1 | 2 | 3 |
|---|---|---|---|
| Tank Size | 35 gallon | | |
| Batch size | 275 lbs | | |
| Emulsification Temperature °C | 75-80 | | 80-85 |
| Order of Addition of the Emulsifiers | Add Cetyl Alcohol Then add Emulsiphos® | Add Emulsiphos® Then add Cetyl Alcohol | |
| **Homogenizer** | **Silverson 375** | | **IKA DR*2000/05** |
| Homogenizer horse power (hp) | 2 | | 10 |
| **Energy Density (hp/lbs/min)** | 0.04 | | 0.22 |
| **Homogenizer # of stator & rotor** | 1 | | 3 |
| Homogenizer generator type | Round Hole | Square Hole | Coarse/Medium/Medium |
| Results | Fail of apperance | Fail of apperance | Pass |

The inventors determined that an energy density at least higher than 4.93 to 8.22 W/kg/s (0.18 to 0.30 hp/lb/min) for three stages of rotor and stator homogenizers resulted in a product having the desired characteristics.

Methods and Results

5

Differential Scanning Calorimeter

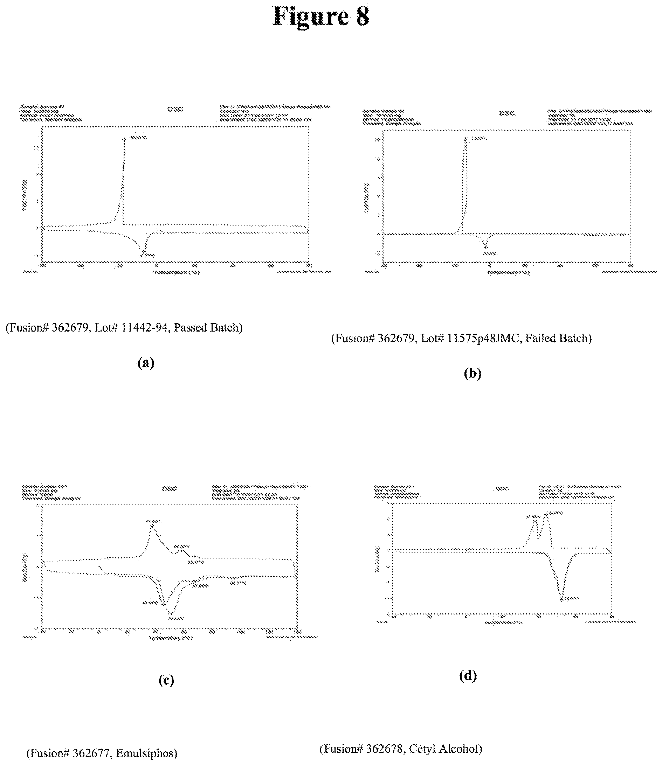

Differential scanning calorimetry (DSC) is a thermoanalytical technique in which the difference in the amount of heat required to increase the temperature of a sample and a reference is measured as a function of temperature. Both sample and reference are maintained at nearly the same temperature throughout the experiment. Generally, the temperature program for a DSC analysis is designed such that the sample holder temperature increases linearly as a function of time. The reference sample should have a well-defined heat capacity over the range of temperatures to be scanned. DSC is used herein to confirm the hydration of fatty alcohol, which in this case is cetyl alcohol. DSC can reveal the hydration of emulsifiers, various intercomponent interactions, and the nature of the binding forces in the gel structure.[10]. Figure 8 shows that a relatively higher peak was observed during heating of cetyl alcohol as compared to Emulsiphos®, which might be due to the fact of less hydration of cetyl alcohol.

FT-IR

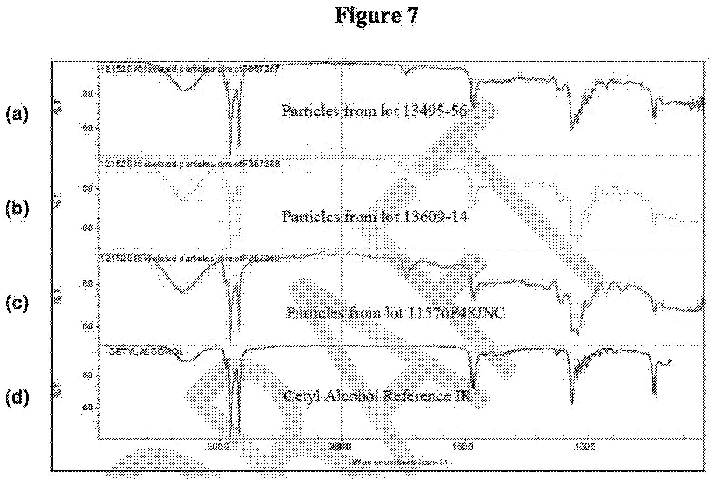

Fourier-transform infrared spectroscopy (FT-IR) is a technique used to obtain an infrared spectrum of absorption or emission of a solid, liquid or gas. An FT-IR spectrometer simultaneously collects high-spectral-resolution data over a wide spectral range. This confers a significant advantage over a dispersive spectrometer, which measures intensity over a narrow range of wavelengths at a time. FT-IR is used to characterize and confirm the components of the white specks. Figure 7 demonstrates that the white specks are confirmed to be crystals of cetyl alcohol.

SEM-EDS

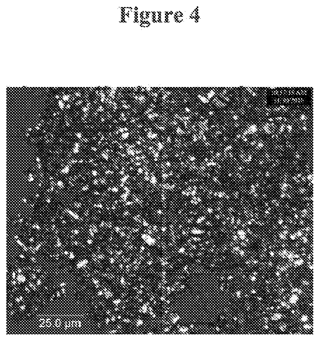

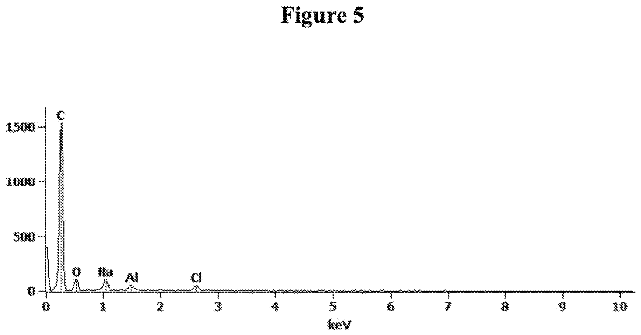

Energy Dispersive X-Ray Spectroscopy (EDS) is a chemical microanalysis technique used in conjunction with scanning electron microscopy (SEM). Energy-Dispersive X-Ray Spectroscopy (EDS) Interaction of an electron beam with a sample target produces a variety of emissions, including x-rays. EDS can be used to find the chemical composition of materials down to a spot size of a few microns, and to create element composition maps over a much broader raster area. A scanning electron microscope (SEM) is a type of electron microscope that produces images of a sample by scanning the surface with a focused beam of electrons. The electrons interact with atoms in the sample, producing various signals that contain information about the sample's surface topography and composition. White specks are mainly composed of cetyl alcohol as confirmed by SEM (see Figure 4 and Figure 5). SEM-elemental analysis revealed that white specks were composed of carbon and oxygen, a trace of sodium and chlorine, but phosphorous was not detected, however which is clearly detected in Emulsiphos®. Therefore, the white specs are confirmed to be cetyl alcohol.

Comparison of Global Homogenizers

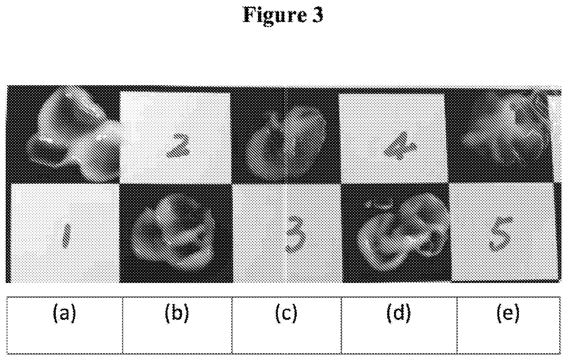

To further understand the importance of homogenization on the formation of the liquid crystal structure, the same process as above except that different homogenizers were employed. Results are captured and summarized in Table 3. Final appearances are shown in Figures 3(a)-3(e). The results demonstrate that a 3-stage rotor and stator and relatively high energy density obtains product with good appearance. It is hypothesized that higher energy obtained from the homogenizer ensures better association of the Emulsiphon® and the cetyl alcohol, thus ensuring successful formation of the liquid crystal structure.

Table 3

| Item | 1 | 2 | 3 | 4 | 5 |
|---|---|---|---|---|---|
| Homogenizer Type | QUADRO Z1 | SILVERSON 375 | EL-Z48 inline dispering | QUADRO Ytron Z3 Emulsifier | Silverson L4RT |
| Homogenizer configuration | 3 stage rotor and stator | 1 stage rotor and stator | 1 stage rotor and stator | 3 stage rotor and stator | 1 stage rotor and stator |
| Energy Density Ev (hp/lb/min) | 0.23 | 0.04 | 0.27 | 0.45 | N/A |
| Results | pass | Fail | fail | pass | pass |

Conclusion

It is confirmed that the white specks came from the aggregates of crystalline cetyl alcohol. The apparent discrepancy between the above results may be because of droplet size reduction through homogenization on the molecular association of fatty alcohols with anionic surfactants.

With regard to the results for Item 5, wherein only 1 stage and rotor was employed, Silverson L4RT lab equipment employs technology that is different than that in pilot and production runs. In this equipment, the pump could be used to control the flow rate of product, which in turn, could control the energy density. The lab instrument basically uses one strong shear energy and appliea it to the small scale amount of lab product.

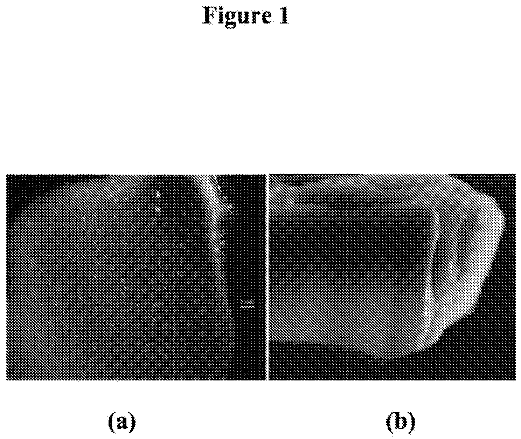

To better ensure the molecular level association between two emulsifiers, a three-stage rotor and stator and high homogenization energy is required. To further prove the hypothesis, another batch was prepared. The appearance of this batch turned out to be acceptable. See Figure 1(b).

It will be understood that, while various aspects of the present disclosure have been illustrated and described by way of example, the invention claimed herein is not limited thereto, but may be otherwise variously embodied according to the scope of the claims presented in this patent application.

## References

1) Ayannides, C.A. et al. (2002) J. Cosmet, Sci., 53: 165-173.

2) Schueller, R et al. (1998) Cosmet. Toiletries, 113: 39-44.

3) Gilsane G. Morais, etc. Influence of Mixing Speed in Liquid Crystal Formation and Rheology of O/W Emulsions containing Vegetable Oils. Journal of Dispersion Science and Technology, 35:1551-1556,2014.

4) Bruna Galdorfini Chiari et al. (December 12th 2012). Cosmetics' Quality Control, Latest Research into Quality Control Isin Akyar, IntechOpen, DOI: 10.5772/51846. Available from: https://www.intechopen.com/books/latest-re search-into-quality-control/cosmetics-quality-control.

5) Yihan Liu et al. Role of liquid crystal in the emulsification of a gel emulsion with high internal phase fraction. Journal of Colloid and Interface Science 340 (2009) 261-268.

6) Jochen Weiss, Emulsion Processing- Homogenization, Food Structure and Functionality Workshop, Department of Food Science and Biotechnology, University of Honeheim, Emulsion Workshop, November 13-14, 2008, Amherst, MA.

7) J. Vilasau etc. Phase behavior of a mixed ionic/nonionic surfactant system used to prepare stable oil-in-water paraffin emulsion. Colloids and Surfaces A; Physicochem. Eng. Aspects 384 (2011) 473-481.

8) Guiillaume Toquer et al., Colloidal shape controlled by molecular adsorption at liquid crystal interface. The Journal of Physical Chemistry 2008, 112, 4157-4160.

9) J.M Ashua, Polymer dispersion: principles and applications.

10) Provost Christine. The application of DSC in the physico-chemical characterization of transparent oil-water gels. Bull. Soc. Chem. Belg. Vol. 98. No. 7 (1989):423-427.

## Claims

1. A process for preparing a liquid crystal structure composition comprising (1) a negatively charged phospholipid and/or phospholipid derivative; and (2) a fatty alcohol, the process comprising using a three-stage rotor and stator homogenizer configuration;
   wherein the negatively charged phospholipid and/or phospholipid derivative is a phospholipid derivative which is potassium cetyl phosphate, hydrogenated palm glycerides; and wherein the fatty alcohol comprises from 8 to 34 carbon atoms and wherein the fatty alcohol is cetyl alcohol.

2. The process of claim 1, further comprising:
   employing an energy of at least 4.93 W/kg/s (0.18 hp/lb/min) for three stages of rotor and stator homogenization.

3. A composition prepared according to the process of claim 1 further comprising:

   a) an emollient, which is cocoglyceride, from >0% to 10%, preferably from 2% to 6%; more preferably from 3% to 6% by weight of the composition;
   b) an emollient wax, preferably a cetyl alcohol, from >0% to 8%, preferably from 1% to 4%; more preferably from 1.5% to 3% by weight of the composition;
   c) an emulsifier, preferably a cetyl phosphate, from 0.2% to 1.4%, preferably from 0.4% to 1.4%; more preferably from 0.5% to 0.6% by weight of the composition;

d) a gelling agent, preferably a carbomer, from 0.4% to 0.6%, preferably from 0.4% to 0.55% by weight of the composition; and
e) from 60% to 90% water by weight of the composition.

## Patentansprüche

1. Verfahren zur Herstellung einer Zusammensetzung mit einer Flüssigkristallstruktur, die (1) ein negativ geladenes Phospholipid und/oder Phospholipid-Derivat und (2) einen Fettalkohol umfasst, wobei das Verfahren die Verwendung einer Konfiguration mit einem dreistufigen Rotor-Stator-Homogenisator umfasst; wobei es sich bei dem negativ geladenen Phospholipid und/oder Phospholipid-Derivat um ein Phospholipid-Derivat handelt, bei dem es sich um Kaliumcetylphosphat, hydrierte Palmglyceride handelt; und wobei der Fettalkohol 8 bis 34 Kohlenstoffatome umfasst und wobei es sich bei dem Fettalkohol um Cetylalkohol handelt.

2. Verfahren nach Anspruch 1, das weiterhin Folgendes umfasst:
die Verwendung einer Energie von mindestens 4,93 W/kg/s (0,18 hp/lb/min) für drei Stufen der Rotor-Stator-Homogenisierung.

3. Zusammensetzung, hergestellt gemäß dem Verfahren nach Anspruch 1, das weiterhin Folgendes umfasst:

   a) >0 % bis 10 %, vorzugsweise 2 % bis 6 %, stärker bevorzugt 3 % bis 6 %, bezogen auf das Gewicht der Zusammensetzung, eines Emolliens, bei dem es sich um Cocoglycerid handelt;
   b) >0 % bis 8 %, vorzugsweise 1 % bis 4 %, stärker bevorzugt 1,5 % bis 3 %, bezogen auf das Gewicht der Zusammensetzung, eines weichmachenden Wachses, vorzugsweise eines Cetylalkohols;
   c) 0,2 % bis 1,4 %, vorzugsweise 0,4 % bis 1,4 %, stärker bevorzugt 0,5 % bis 0,6 %, bezogen auf das Gewicht der Zusammensetzung, eines Emulgators, vorzugsweise eines Cetylphosphats;
   d) 0,4 % bis 0,6 %, vorzugsweise 0,4 % bis 0,55 %, bezogen auf das Gewicht der Zusammensetzung, eines Geliermittels, vorzugsweise eines Carbomers; und
   e) 60 % bis 90 %, bezogen auf das Gewicht der Zusammensetzung, Wasser.

## Revendications

1. Procédé de préparation d'une composition à structure à cristaux liquides comprenant (1) un phospholipide et/ou dérivé de phospholipide chargé négativement ; et (2) un alcool gras, le procédé comprenant l'utilisation d'une configuration d'homogénéisateur rotor et stator à trois étages ; dans lequel le phospholipide et/ou dérivé de phospholipide chargé négativement est un dérivé de phospholipide qui est le phosphate de cétyle de potassium, des glycérides de palme hydrogénés ; et dans lequel l'alcool gras comprend de 8 à 34 atomes de carbone et dans lequel l'alcool gras est l'alcool cétylique.

2. Procédé selon la revendication 1, comprenant en outre :
l'emploi d'une énergie d'au moins 4,93 W/kg/s (0,18 hp/lb/min) pour trois étages d'homogénéisation à rotor et stator.

3. Composition préparée selon le procédé de la revendication 1 comprenant en outre :

   a) un émollient qui est le coco-glycéride, de > 0 % à 10 %, de préférence de 2 % à 6 % ; plus préférablement de 3 % à 6 % en poids de la composition ;
   b) une cire émolliente, de préférence un alcool cétylique, de > 0 % à 8 %, de préférence de 1 % à 4 % ; plus préférablement de 1,5 % à 3 % en poids de la composition ;
   c) un émulsifiant, de préférence un phosphate de cétyle, de 0,2 % à 1,4 %, de préférence de 0,4 % à 1,4 %, plus préférablement de 0,5 % à 0,6 % en poids de la composition ;
   d) un agent gélifiant, de préférence un carbomère, de 0,4 % à 0,6 %, de préférence de 0,4 % à 0,55 % en poids de la composition ; et
   e) de 60 % à 90 % d'eau en poids de la composition.

# Figure 1

(a)                                                        (b)

## Figure 2

| (a) | (b) | (c) | (d) |

## Figure 3

| (a) | (b) | (c) | (d) | (e) |
|-----|-----|-----|-----|-----|

Figure 4

Figure 5

# Figure 6

## Figure 7

# Figure 8

(Fusion# 362679, Lot# 11442-94, Passed Batch)

**(a)**

(Fusion# 362679, Lot# 11575p48JMC, Failed Batch)

**(b)**

**(c)**

**(d)**

(Fusion# 362677, Emulsiphos)

(Fusion# 362678, Cetyl Alcohol)

Figure 9

Figure 10

LIQUID CRYSTALLINE STRUCTURES

a. double layers of emulsifier and fatty alcohols

b. interlamellar fixed water

c. in case of a surplus of lipophilic fatty alcohol a lipophilic gel phase may be formed

d. free water

e. dispersed oil phase

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017074892 A

**Non-patent literature cited in the description**

- **AYANNIDES, C.A. et al.** *J. Cosmet, Sci.*, 2002, vol. 53, 165-173
- **SCHUELLER, R et al.** *Cosmet. Toiletries*, 1998, vol. 113, 39-44
- **GILSANE G. MORAIS**. Influence of Mixing Speed in Liquid Crystal Formation and Rheology of O/W Emulsions containing Vegetable Oils. *Journal of Dispersion Science and Technology*, 2014, vol. 35, 1551-1556
- **BRUNA GALDORFINI CHIARI et al.** Cosmetics' Quality Control, Latest Research into Quality Control Isin Akyar. *IntechOpen*, 12 December 2012, https://www.intechopen.com/books/latest-research-into-quality-control/cosmetics-quality-control
- **YIHAN LIU et al.** Role of liquid crystal in the emulsification of a gel emulsion with high internal phase fraction. *Journal of Colloid and Interface Science*, 2009, vol. 340, 261-268
- **JOCHEN WEISS**. Emulsion Processing- Homogenization, Food Structure and Functionality Workshop, Department of Food Science and Biotechnology. *Emulsion Workshop*, 13 November 2008
- **J. VILASAU**. Phase behavior of a mixed ionic/nonionic surfactant system used to prepare stable oil-in-water paraffin emulsion. *Colloids and Surfaces A; Physicochem. Eng. Aspects*, 2011, vol. 384, 473-481
- **GUIILLAUME TOQUER et al.** Colloidal shape controlled by molecular adsorption at liquid crystal interface. *The Journal of Physical Chemistry*, 2008, vol. 112, 4157-4160
- **PROVOST CHRISTINE**. The application of DSC in the physico-chemical characterization of transparent oil-water gels. *Bull. Soc. Chem. Belg.*, 1989, vol. 98 (7), 423-427